# EUROPEAN PATENT APPLICATION

(11) **EP 1 285 628 A2**
(43) Date of publication of application: **26.02.2003**
(21) Application number: 02018581.5
(22) Date of filing: 19.08.2002
(51) Int. Cl.: A61B 5/15

(54) **Capillary device for sampling and transfer of fluids**

(30) Priority: 22.08.2001 US 934865
(71) Applicant: Becton Dickinson and Company, Franklin Lakes, New Jersey 07417-1880 (US)
(72) Inventor: Niermann, Volker, Little Falls, New Jersey 07242 (US)
(74) Representative: von Kreisler, Alek, Dipl.-Chem.

(57) **Abstract**

A fluid transfer device collects fluid by capillary attraction into a housing and provides for the subsequent dispensing of the collected fluid. The fluid transfer device includes a housing defining a housing interior, and including an access port and elongate capillary channel in fluid communication with the access port and the housing interior. Fluid is drawn through the access port and into the capillary channel under capillary action. The housing is deformable so as to compress the housing interior and exert an expelling force on the capillary channel to expel the fluid from the access port.

## Description

### BACKGROUND OF THE INVENTION

### 1. Field of the Invention

The present invention is directed to a fluid transfer device which collects fluid by capillary attraction into a housing and provides for the subsequent dispensing of the collected fluid. More particularly, the present invention is directed to a device for transferring small blood samples from a patient to a laboratory chip for analysis or to a point of care testing cartridge.

### 2. Description of Related Art

Small blood samples such as those from a patient's finger stick are typically collected with a device such as a small glass capillary tube. The blood is attracted into the tube under capillary action. The blood-filled tube is then transported to an analysis device where the blood sample is expelled from the tube so that analytical procedures may be performed on the collected sample. Typically, the blood sample is deposited on a small chip-like substrate which is compatible with the particular analytical device employed or to a point of care testing cartridge.

In order to expel the collected blood sample on the chip, the medical technician must employ a separate syringe-like component. The syringe is placed at one end of the capillary tube and is manually actuated to expel the blood from the other end of capillary tube. As may be appreciated, this expelling technique is cumbersome and time consuming. Furthermore, it requires the technician to employ a separate component such as syringe. In practice, the medical technician must insert the thin plunger of the syringe into the capillary tube of the collection device and depress the plunger to expel the blood within the capillary tube. This operation requires a high degree of manual dexterity, necessitating that the operator hold the capillary tube with one hand and the syringe with the other hand while simultaneously depressing the plunger. The two handed operation required for expelling the collected blood sample from the capillary tube prevents the technician from holding steady the analysis chip onto which the blood is dispensed.

Another disadvantage of the capillary tube is that it is often difficult to obtain and dispense an accurate blood sample amount. Due to capillary action, blood will be continually drawn into the tube. As a result, the accurate dispensing of a quantity of the blood sample onto the chip is difficult to achieve using the available components.

Furthermore, the capillary tube itself is typically formed of glass. Breakage of the glass capillary tube after blood collection poses a serious biohazard risk to the technician. While the glass capillary tube may be wrapped or contained in a plastic package, in an effort to minimize such biohazard, the problems with glass breakage cannot be entirely avoided. Even after successful depositing of the sample on the chip, the expended glass capillary tube still presents a potential biohazard as it must be transported to disposal. Further, where the tube is not damaged, there is the risk of improperly reusing the tube with another patient.

It is, therefore, desirable to provide a blood collection and transfer device which can be easily manipulated by the technician and which presents no hazard of biocontamination due to glass breakage.

### SUMMARY OF THE INVENTION

The present invention provides a fluid transfer device for collecting and transporting fluids such as a blood sample from, for example, a patient to a chip or to a point of care testing cartridge for laboratory analysis.

The fluid transfer device of the present invention includes a housing, defining a housing interior and including an access port with an elongate capillary channel in fluid communication with the access port and the housing interior. The capillary channel draws fluid through the access port into the capillary channel under capillary action. The housing is deformable so as to compress the housing interior and exert an expelling force on the capillary channel to expel the fluid from the access port.

In one embodiment of the present invention, the housing includes an interiorly deformable contoured dimple defining the housing interior. Deformation of the dimple causes compression of the housing interior. The dimple is non-resiliently deformable, preventing reuse of the fluid transfer device.

The capillary channel may follow a tortuous path within the housing for restricting the amount of fluid drawn into the device. The transfer device may also support fluid stop along the channel, limiting the amount of fluid drawn into the channel.

The housing may also include a view window in visual communication with the capillary channel so that a visual indication is provided as to the proper amount of fluid draw into the fluid transfer device.

The housing interior, including the dimple, may be constructed so that a precise, pre-determined amount of fluid may be collected and dispensed from the device.

### DESCRIPTION OF THE DRAWINGS:

FIG. 1 is a perspective showing of the fluid transfer device of the present invention positioned over a laboratory analysis chip.

FIG. 2 is a perspective showing of a further embodiment of the fluid transfer device of the present invention.

FIGS. 3 and 4 are perspective showings of the components used to form the fluid transfer device of FIG. 1.

FIG. 5 is a vertical sectional view of the fluid transfer device of FIG. 1 taken through the lines 5 - 5 thereof.

### DETAILED DESCRIPTION OF THE INVENTION

The present invention provides a fluid transfer device for collecting fluids under capillary action, transferring fluids to a remote site, and expelling fluids from the device under an expelling force. While the device of the present invention may be used to collect, transport and expel any type of fluid, the present invention has a particular utility in the medical analytical field, for example, for obtaining a blood sample from a patient, transporting that blood sample to a remote location where it may be dispensed onto a laboratory analysis chip compatible with a conventional analytical device.

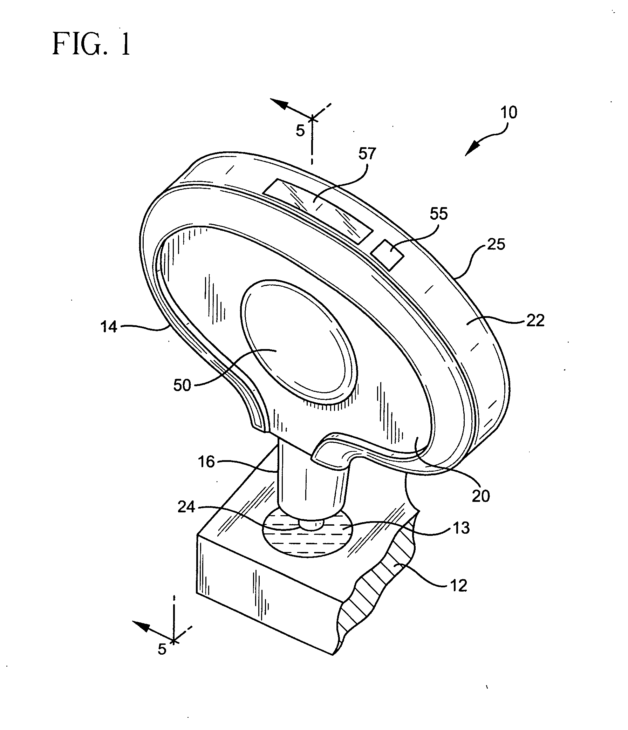

Referring now to FIG. 1, the fluid transfer device **10** of the present invention is shown positioned over a laboratory analysis chip **12** onto which a preselected amount of a fluid sample **13** can be deposited. As described above, the present invention provides a particular utility with regard to collecting a patient's blood sample from, for example, a needlestick and transferring the blood sample to a chip which is compatible with a blood analysis device. While not limited to collecting blood samples, the present invention will be described with reference thereto.

The fluid transfer device of FIG. 1 is generally a bulb-shaped member having a main body **14** and a collection/dispensing tip **16** extending therefrom. Main body **14** is generally oval in shape having a pair of spaced-apart planar surfaces **18** and **20**, and a perimetrical sidewall **22** extending therebetween. Collection/dispensing tip **16** depends from sidewall **22** and is generally cylindrical in shape, having a narrowing distal tip end **24** extending therefrom.

As will be described in further detail hereinbelow, fluid transfer device **10** may be formed from a pair of conventionally formed plastic components. Any of a variety of plastic forming techniques such as injection molding may be employed to form fluid transfer device **10**. For example, the components forming fluid transfer device may be molded polyolefin or polystyrene.

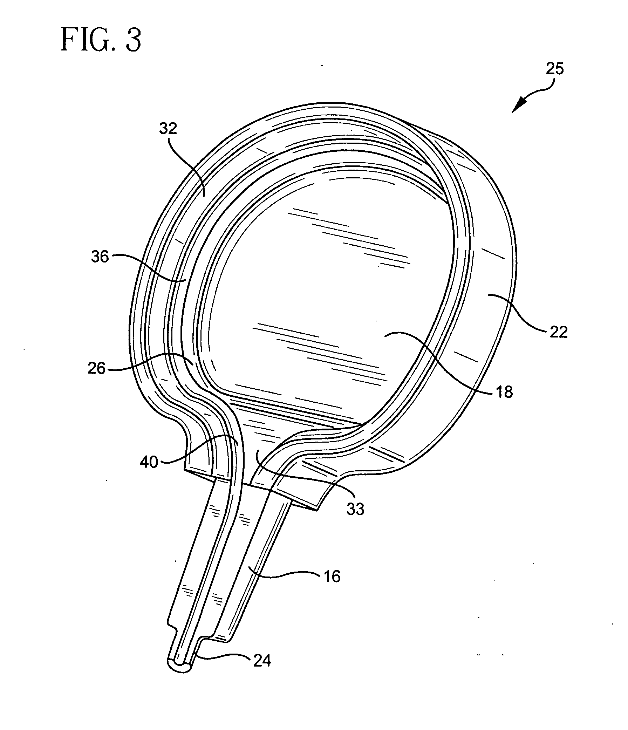

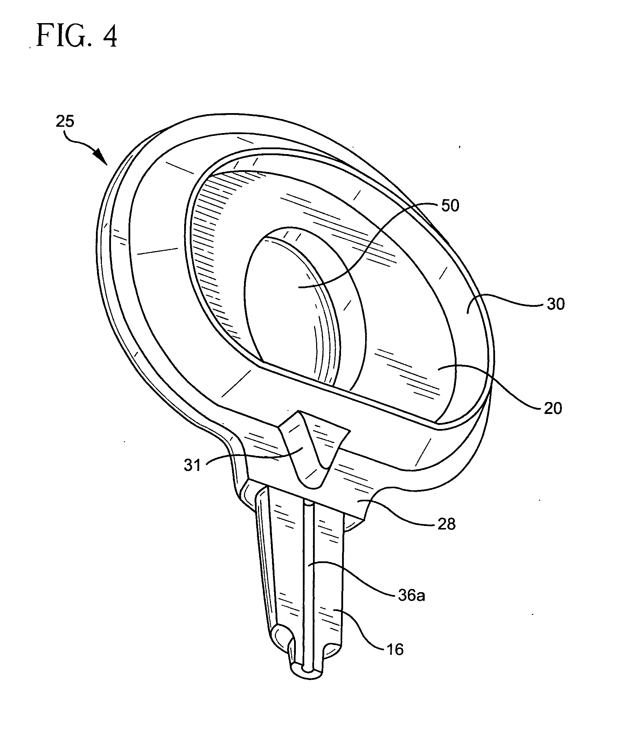

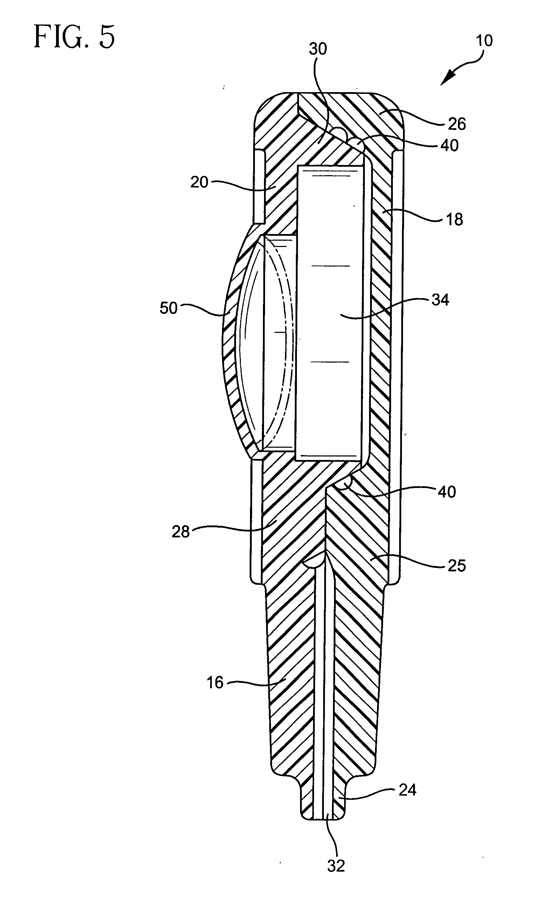

Referring additionally to FIGS. 3, 4 and 5, it can be seen than the fluid transfer device is defined by a two-part housing **25** wherein a first part **26** accommodating therein a second part **28** so as to form housing **25**. First part **26** includes planar surface **18** with sidewall **22** extending outwardly about the periphery thereof. Similarly, second part **28** includes planar surface **20** with an outwardly projecting skirt **30** extending from just inward of the periphery thereof. Skirt **30** interfits with an interior surface **32** of sidewall **22** so as to define a housing interior **34** between planar surfaces **18** and **20.** Further, first and second parts **26** and **28** of housing **25** define one-half of collection/dispensing tip **16** so that when assembled as shown in FIG. 5, a cylindrically configured tip is formed.

First part **26** of housing **25** is provided with an elongate trough-shaped track **36** formed on the interior surface **32** of sidewall **22.** Track **36** extends from the distal extent of tip end **24** of collection/dispensing tip **16** and extends through tip **16** and into and around the interior surface **32** of sidewall **22**. Track **36** may extend any desirable length around sidewall **22** and preferably extends in spiral fashion therearound. At the collection/dispensing tip **16** a mating track **36a** may be formed in second part **28** so that when assembled a continuous channel is provided to tip end **24.**

The first part **28** is assembled to second part **26** in a frictional or snap fit fashion. To facilitate such attachment, second part **28** may include a projection **31** at the junction of planar surface **20** and tip **16** which is fitted into a recess **33** in first part **26**.

With first part **28** assembled to second part **28** as shown in FIG. 5, track **36** defines a spiral capillary channel **40** which extends from tip end **24** defining an access port **32** thereat, to an opposite end which is in communication with the interior **34** of housing **25**. Such communication with the interior **34** of housing **25** may be provided by extending capillary channel **40** in an inwardly spiraling fashion until the track formed in sidewall **22** communicates with the interior **34** by extending beyond the skirt **30** extending from planar surface **18**. Thus, in an assembled position as shown in FIG. 5, an elongate small-diameter capillary channel **40** is formed between access port **32** at tip end **24** and the interior **34** of housing **25.**

Owing to this small diameter of capillary channel **40,** the fluid transfer device **10** functions as a capillary tube enabling fluid such as blood from a fingerstick to be drawn into the interior **34** of housing **25** by capillary attraction. As will be described hereinbelow, the length and diameter of the capillary channel **40** may be selected so as to collect a given amount of fluid sample.

The interior **34** of housing **25** is further defined by an outwardly extending, inwardly deformable contoured dimple **50** on planar surface **18**. Outwardly extending dimple **50** further defines the interior volume of housing **25**. Dimple **50** is interiorly depressible so as to reduce the interior volume of housing **25.** The materials forming fluid transfer device **10** are such that the dimple **10** is nonresiliently deformable so that, once collapsibly deformed, it remains in its collapsed position. This prevents the re-use of fluid transfer device **10** as, once collapsed, the housing can no longer be used as a capillary device.

Having described the basic components of a fluid transfer device of the present invention, its operation may now be described.

Referring to FIG. 1, fluid transfer device **10** of the present invention may be placed over a needlestick placed in the patient's finger (not shown). The tip end **24** is placed over the blood site and blood is attracted or "wicked" into the capillary channel **40** through collection/dispensing tip **16.** As is well known, the capillary attraction of blood sample **13** may be enhanced by venting which is provided in housing **25** in communication with channel **40**. The blood will continue to be wicked into device **10** until it is met by sufficient resistance. The amount of blood that is attracted under capillary action and collected in housing **25** may be selected by providing a capillary channel of certain length and diameter. The selected length and diameter of the channel dictates the amount of blood drawn into the interior **34** of housing **25.** It is also contemplated that, in addition to the diameter and length of the capillary channel, the path traversed by the capillary channel along housing **25** may be selected so as to also restrict the amount of fluid flow into capillary channel **40**. As the channel includes a more tortuous path, it is more difficult to attract blood therethrough as blood flow is met by resistance of the channel shape. This limits the amount of blood collected by device **10**.

It is further contemplated that techniques for interrupting flow of fluid around the capillary channel may be also provided so as to limit the amount of blood collected. For example, various devices may be incorporated into housing **25** in communication with capillary channel **40** so as to provide resistance to the flow of fluid therethrough. One such flow resistance device **55**, shown schematically in FIG. 1, may include a one-way valve. Similarly, textile materials such as cotton may be used or a hydrophobic material such as polyethylene terephthalate, glycol modified. These devices provide resistance to flow in the capillary channel and, therefore, restrict the amount of fluid which will be drawn into channel.

It is further contemplated that, in order to assure that the proper amount of fluid has been drawn into housing **25**, the sidewall **22** may include a transparent or translucent viewing window **57** in visual communication with the channel **40.** This provides a visual indication of proper fluid draw into the fluid transfer device. This transparent window may be used in combination with the forming of device **10** of frosted material which also provides some degree of internal viewability.

Upon collection of the proper amount of blood drawn into the fluid transfer device **10**, it may be transported to a position over analysis chip **12**.

Once positioned, the technician can inwardly press deformable dimple **50** so as reduce the volume of interior **34.** Dimple **50** may be manually depressed under manual pressure to a position shown in dotted lines in FIG. 5. The size, location and depth of dimple **50** provide controlled air pressure upon channel **40**. This effects displacement pressure within the housing interior **34**. Such displacement pressure exerts an expelling hydraulic force on the blood retained in capillary channel **40**. Since capillary channel **40** is in communication with the interior **34** of housing **25,** the expelling force tends to expel the blood sample **13** through tip end **24** of tip **16** and on to analysis chip **12.** As the inward depression of dimple **50** reduces the interior volume of housing **25** a pre-selected known quantity of the blood sample will be expelled onto laboratory chip **12.**

In order to prevent the sample from being expelled in a quick and uncontrolled manner, the displacement pressure applied by the depression of dimple **50** may be restricted by a pressure reducing valve such as an orifice plate near flow resistance device **55**. This allows controlled, smooth expelling of the blood sample, preventing uncontrolled dispensing therefrom.

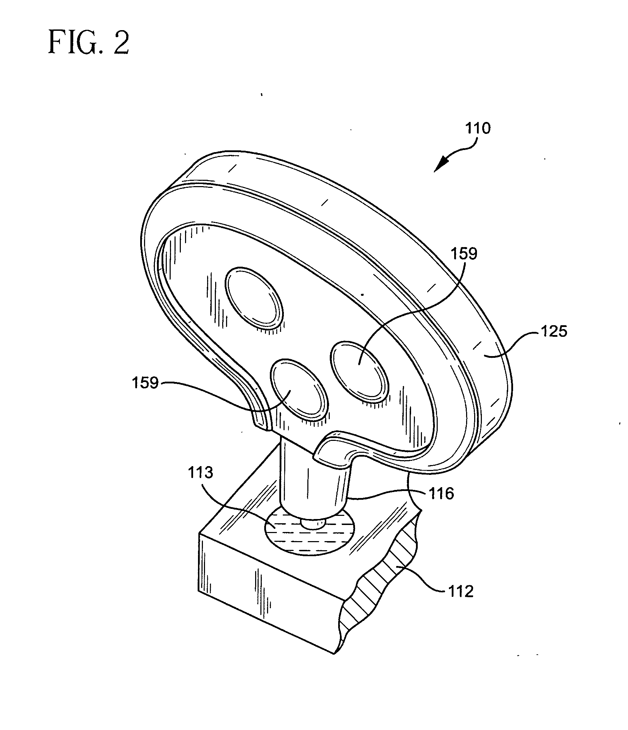

Various other techniques may be used to control the amount of blood expelled from fluid transfer device **10**. For example, as shown in FIG. 2, a fluid transfer device **110** includes a housing-**125** and a dispensing/collecting tip **116.** A planar surface **120** of housing **125** includes plural dimples **159**. Each dimple would reduce the interior fluid transfer device a given amount so as to exert a pre-selected hydraulic expelling force on capillary channel **40**. In this manner, the number of dimples depressed would dictate the amount of fluid sample **113** expelled from the fluid transfer device onto chip **112**. Further, the shape and size of the dimples could be selected so as to alter the amount of fluid expelled from the fluid transfer device.

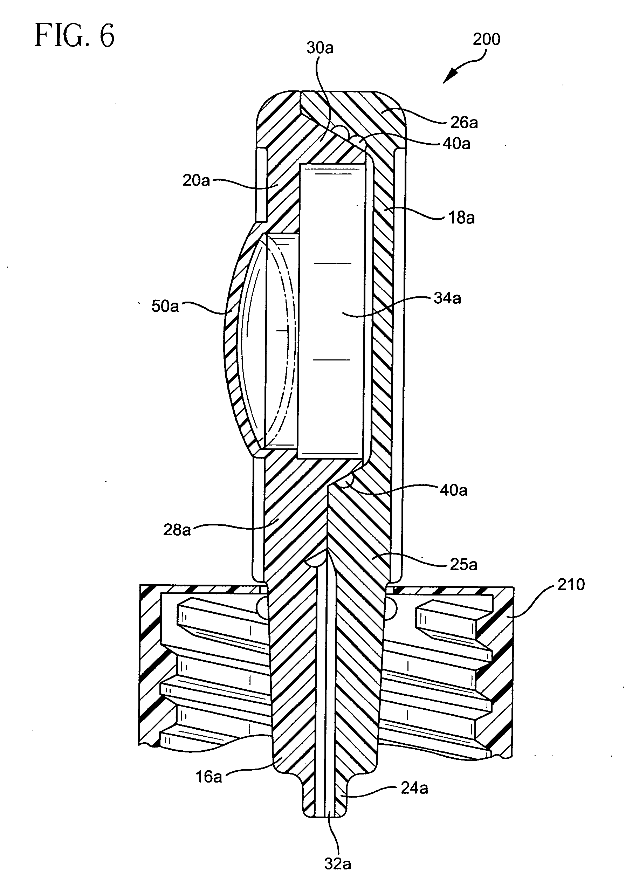

The invention, as shown in FIG. 6 includes many components which are substantially identical to the components of FIGS. 1-5. Accordingly, similar components performing similar functions will be numbered identically to those components of FIGS. 1-5, except that a suffix "a" will be used to identify those similar components in FIG. 6.

Fluid transfer device **200**, as shown in FIG. 6 is an alternate embodiment of the present invention. As shown in FIG. 6, device **200** includes a permanent or removably attached male luer lock connection **210** on dispensing tip **16a** for coupling to a female luer lock which may be permanently or removably connected to a laboratory chip or point of care testing cartridge.

Alternatively, it is within the purview of the invention that the removably attached or permanently attached male luer lock connection is on the laboratory chip or point of care cartridge and the female luer lock is permanently attached or removably connected on dispensing tip **16a.** The male and female luer locks can be any of the many various combinations that are known or any other adaptor systems that can be readily fitted to the device of the present invention and the relating laboratory chip, point of care testing cartridge and the like.

Various other changes and modifications may be effected therein by one skilled in the art without departing from the scope or spirit of the invention, and it is intended to claim all such changes and modifications as fall within the scope of the invention.

## Claims

1. A fluid transfer device comprising:
a housing defining a housing interior and including an access port and an elongate capillary channel in fluid communication with said access port and said housing interior for drawing fluid through said access port and into said capillary channel under capillary action;
said housing being deformable so as to compress said housing interior and exert an expelling force on said capillary channel to expel said fluid from said access port.

2. A fluid transfer device of claim 1 wherein said housing transfer includes:
a bulb shaped member defining said housing interior; and
a collection/dispensing tip including said access port, said capillary channel extending from said bulb shaped member to said access port.

3. The fluid transfer device of Claim 1, wherein said housing includes an interiorly deformable contoured dimple further defining said housing interior, deformation of said dimple causing said housing interior compression.

4. The fluid transfer device of Claim 3, wherein said dimple is non-resiliently deformable.

5. The fluid transfer device of Claim 1, wherein said device includes said capillary channel being formed about the perimeter of said housing and said housing being deformable within said perimeter.

6. The fluid transfer device of Claim 5, wherein said access port is located adjacent said housing perimeter in communication with one end of said capillary channel.

7. The fluid transfer device of Claim 6, wherein said other end of said capillary channel is in fluid communication with said housing interior.

8. The fluid transfer device of Claim 7, wherein said capillary channel includes means for restricting fluid drawn therethrough.

9. The fluid transfer device of Claim 8, wherein said draw restricting means includes a fluid stop supported by said channel intermediate said housing interior and said access port.

10. The fluid transfer device of Claim 9, wherein said stop includes a fluid valve.

11. The fluid transfer device of Claim 5, wherein said housing includes a first housing member and second housing member attachable to said first housing member to define therebetween said housing interior, wherein said first housing member includes an extending sidewall housing with said capillary channel being formed therein, and wherein said second housing member includes an extending skirt engageable with said sidewall for securing said first housing member to said second housing member.

12. The fluid transfer device of Claim 5, wherein said housing includes a view window in visual communication with said capillary channel.

13. The fluid transfer device of Claim 1, wherein said housing includes a plurality of interiorly deformable dimples defining said housing interior, deformation of dimples causing said depression of said housing interior and explosion of a pre-determined quantity of said fluid from said access port.

14. The fluid transfer device of Claim 1, further comprising a removably attached male luer lock or a removably attached female luer lock, or a male luer lock, or a female luer lock on said access port.
